# EUROPEAN PATENT SPECIFICATION

(11) **EP 1 600 461 B1**
(45) Date of publication and mention of the grant of the patent: **12.12.2012**
(21) Application number: 04012228.5
(22) Date of filing: 24.05.2004
(51) Int. Cl.: C08B 37/00, A23F 5/48, A23L 1/22

(54) **Arabinogalactan isolate from green and roasted coffee for food and delivery applications and process for its production**
Arabinogalaktan extrahiert aus Röst- und Rohkaffee für Nahrungs- und Abgabe-Anwendungen und Verfahren zu deren Herstellung
Arabinogalactane isolé à partir de café vert ou torrifié aux fins d'applications alimentaires et de délivrance et procédé de préparation.

(43) Date of publication of application: 30.11.2005
(73) Proprietor: Nestec S.A., 1800 Vevey (CH)
(72) Inventor: Curti, Delphine Gisele, 1042 Bettens (CH); Gretsch, Catherine, 1412 Valeyres-sous-Ursins (CH); Labbe, David Philippe, 1010 Lausanne (CH); Redgwell, Robert John, 1073 Savigny (CH); Schoonman, Johanna Hendrika, 1820 Montreux (CH); Ubbink, Johan Bernard, La Claie-aux-Moines 1073 Savigny (CH)

(56) References cited:
- WO-A-96/09773
- US-A- 3 845 220
- REDGWELL R J ET AL: "Coffee bean arabinogalactans: acidic polymers covalently linked to protein" CARBOHYDRATE RESEARCH, ELSEVIER SCIENTIFIC PUBLISHING COMPANY. AMSTERDAM, NL, vol. 337, no. 3, 11 February 2002 (2002-02-11), pages 239-253, XP004335950 ISSN: 0008-6215

## Description

### Field of the invention

The sensory quality of foodstuffs are, next to the nutritional value and safety, the most important factor determining the consumer acceptance of a food product. For example, in addition to their physiological effects, coffee beverages are appreciated for their sensory characteristics. The most important of these sensory characteristics are aroma and taste of the coffee, but others, like the mouthfeel of the beverage and its visual appearance, are also of significance.

In soluble coffee, it is often attempted to re-create the sensory characteristics of a freshly produced roast & ground coffee as faithfully as possible. Occasionally, in the development and production of soluble coffee, the principal focus is to develop sensory characteristics different from a traditional roast & ground coffee, but the aim is invariably to optimise the sensory profile of the soluble coffee in such a way that consumer preference is best satisfied.

The sensory characteristics of soluble coffee are dependent in a complex way on the coffee blend used in its manufacture, its roasting conditions, the drying technology, the storage conditions of the powder, and the way the soluble coffee is prepared by the consumer. Current soluble coffee developments are facilitated because, nowadays, for some of the sensory characteristics of soluble coffee, correlations are well-established between the sensory characteristics and chemical, structural and physical properties of the soluble coffee.

For instance, it is known that the mouthfeel of a beverage is enhanced by tuning the viscosity of the beverage to its optimal value, which is both not too high and not too low. It is general rheological knowledge that the viscosity of a solution or dispersion is mainly effected by its high-molecular weight constituents, in foods often denoted as hydrocolloids and often consisting of carbohydrates.

The relation between the sensory characteristics and the relevant physical, structural and chemical parameters is not always so simple as in case of the mouthfeel of a beverage, even though this relation can still be quantitatively specified in many of these more complex cases. For instance, the important sensory characteristic of the aroma of a soluble coffee beverage is the result of the impact of a complex but balanced mixture of about 800 volatile compounds on the olfactory epithelium. Many physico-chemical and sensory properties of these 800 compounds are known and it is also known how they contribute to the character of coffee aroma. The volatile aroma compounds are largely formed during the roasting process and are partially incorporated in the final soluble coffee powder. During preparation of the soluble coffee beverage, the coffee powder dissolves, and the aroma compounds are released to the olfactory epithelium via a number of intermediate steps.

One of the key problems experienced with soluble coffee, such as pure soluble coffee, is that its aroma strength and quality diminish with storage time of the powder. This loss of aroma strength and quality manifests itself even during the storage of soluble coffee under close-to-optimal storage conditions (a low moisture content and ambient temperature) and during the common shelf life of a soluble coffee, usually one year up to 3 years. However, it is gravely worsened under adverse storage conditions like high moisture levels or elevated temperatures.

It is known that sensitive aromas can be protected by encapsulation or entrapment in a glassy carbohydrate matrix. This protection is generally not complete, and the degree of protection may vary from one aroma compound to another one, but the overall impact of such so-called glass encapsulation of aromas is that its quality is perceivably better preserved than of non-encapsulated aromas. Glass encapsulation of aromas is invariably achieved using carbohydrates in the amorphous state.

In order to improve many of the important sensory characteristics of pure soluble coffee, like its freshness, it would be a general strategy to employ certain types of carbohydrates, which either optimise the viscosity of the beverage or may be used to protect the coffee aroma during storage. Because of regulatory requirements, in the manufacture of pure soluble coffee, such carbohydrates can not originate from generally accepted vegetable sources like starch, but they should be derived from coffee itself. Surprisingly, the inventors have found that such carbohydrates can indeed be extracted from coffee and can be used for the stated purposes.

In conclusion, there still exists a clear need for methods which allow the optimisation of the sensory characteristics of soluble coffee, in particular its mouthfeel and aroma impact and quality including its aroma freshness. Of major importance is that these methods should be allowed in the manufacture and marketing of soluble coffee. Therefore, any method by which useful carbohydrates can be extracted in relatively pure form from coffee is of major interest for the creation of novel types of foodstuffs, in particular soluble coffee, with enhanced sensory characteristics.

### Prior art

In WO 9955736 and US 2001/0000486 A1 derivatised arabinogalactans with improved rheological, pH, and viscosity profiles are claimed. The derivatisation induces chemical changes which are undesirable from the food perspective.

WO 0044238 relates to an aromatised soluble creamer powder. The application discloses a soluble creamer powder comprising a matrix including an aroma system comprising coffee aroma components and a stabilising amount of soluble coffee solids. The description further discloses that aqueous aroma components are stabilised by adding a suitable amount of coffee solids to them. As ingredients of non-coffee origin are used, the aromatised soluble creamer powder can clearly not be used in pure soluble coffee. In addition, it is surprising to learn that coffee solids are added, as they will provoke degradation of the coffee aroma.

WO 0025606 relates to solid delivery systems for aroma ingredients, which can be obtained by extruding sugar or sugar derivatives or mixtures thereof, including among others arabinose or galactose. As ingredients of non-coffee origin are used, the delivery system can clearly not be used in pure soluble coffee.

In patent application WO200226055 beverage compositions comprising arabinogalactan and vitamins are claimed. Coffee is cited as a source of arabinogalactans but no examples are given of its extraction or use. Arabinogalactans are mentioned as health-promoting ingredient which do not much increase the viscosity of the beverage. However, we have surprisingly shown that coffee-derived arabinogalactans have unusual rheological properties including viscosity enhancement. In addition, the methods described in this application will not enable the extraction of arabinogalactans from coffee with the required molecular weight.

In US 5882520, aqueous two-phase systems are discussed in which at least one of the phases contains arabinogalactan. The arabinogalactans are not of coffee origin and the stated purpose, namely the extraction of biological materials, falls outside the scope of our invention.

US 6271001 discusses gums isolated from plant cell cultures. This patent deals exclusively with polymers from plant cell cultures without establishing the chemical nature or the composition of the plant cell gums. We do not apply plant cell cultures as source of carbohydrates, but extract the arabinogalactan from a plant fruit, namely the coffee bean.

In patent application WO2002041928 a biomaterial useful as controlled-release carrier is claimed. The biomaterial comprises a porous polymer gel matrix in the pores of a hydrophilic or amphiphilic polymer matrix. Arabinogalactan is mentioned as a possible constituent of the gel matrix. However, we do not use such bi-phasic encapsulation matrices.

In US 6296890 a heavily foaming coffee fraction as well as a process for its manufacture are claimed. This fraction is extracted from roasted beans in hot-water followed by precipitations. It is not well characterised chemically and consists of 60% polysaccharide and 40% melanoidin type compounds. For purposes aiming at the improvement of the sensory characteristics of soluble coffee it is not sufficiently well-defined from a rheological point of view and for aroma stabilization it is not useful because of its high content of melanoidin-type compounds.

In summary, none of the prior art discusses a) the extraction by enzymatic means of arabinogalactans from green or roasted coffee and b) its subsequent use as enhancer of the characteristics of food, in particular soluble coffee. =

US-A-3 845 220 discloses a process for extracting coffee comprising enzymatically hydrolysing whole parched coffee beans thereby obtaining an aqueous dispersion comprising partially hydrolysed coffee beans and extracted substances which is further submitted to a centrifugation step to separate and remove the insoluble matter and beans. The nature of the products extracted from the coffee beans is not specified.

REDGWELL R J ET AL ("Coffee bean arabinogalactans: acidic polymers covalently linked to protein", Carbohydrate research 337 (2002) 239-253) disclose a procedure for the isolation of arabinogalactans (=AG) from the cell wall material of coffee beans.

### Brief description of the invention

The present invention pertains to a novel process of obtaining arabinogalactans from green or roasted coffee, the use of this arabinogalactan as an enhancer of the sensory qualities of food, in particular the mouthfeel of soluble coffee and the strength and quality of aroma in soluble coffee after prolonged storage, and a solid glassy matrix comprising coffee aroma, said matrix comprising coffee-derived arabinogalactans, a pure soluble coffee composition and a soluble coffee beverage composition containing the solid glassy matrix.

Green coffee and roasted coffee are known to contain arabinogalactans but up to now the isolation of these arabinogalactans was not possible without inducing excessive degradation of the arabinogalactan. Excessive degradation of the arabinogalactan reduces its molecular weight and leads to chemical modifications which render its application in accordance to the present invention, i.e. as a viscosity modifier and encapsulation matrix, useless.

The present enzymatic process surprisingly isolates arabinogalactan-protein from green and roasted coffee beans which has controlled molecular weight and which has been minimally degraded. These extracted arabinogalactans turn out to be particularly useful to enhance the sensory properties of food, such as beverages and especially soluble coffee, and can be beneficially used as encapsulation matrix for the delivery of sensitive active ingredients, such as aromas in soluble coffee.

The process of the present invention refers to the extraction of arabinogalactans from coffee comprising enzymatically hydrolysing whole or ground green or roasted coffee beans thereby obtaining an aqueous dispersion comprising partially hydrolysed coffee beans and arabinogalactans.

The process further comprises the steps:
a) treating the aqueous dispersion to yield an aqueous solution comprising arabinogalactans, or optionally a powder enriched in arabinogalactans;
b) isolating the arabinogalactans by concentrating the aqueous solution and precipitating the arabinogalactan, thereby obtaining a dispersion of extracted arabinogalactans.

After the extraction and isolation, the properties of the arabinogalactan may be further modified for the final application, for example viscosity control to improve the mouthfeel of a coffee beverage, or the use as encapsulation matrix for coffee aroma, if necessary, by carefully hydrolysing the arabinogalactan to the desired molecular weight profile.

Surprisingly, we have observed that the arabinogalactan from green and roasted coffee obtained in this way is particularly useful to optimise important sensory characteristics of food, e.g. beverages and particular soluble coffee and pure soluble coffee. Novel properties include the unusual rheological behaviour and the ability to form glassy matrices for encapsulation of sensitive active ingredients, like for instance coffee aroma.

### Detailed description of the invention

Arabinogalactans are a family of polysaccharides (proteoglycans) implicated in plant growth and development. They are a family of proteoglycans found in higher plants, occurring in many different tissues: on the plasma membrane, in the cell wall and in the extracellular matrix. The most common source is the Larch tree (*Larix sp.*)*.* Larch arabinogalactan is approved by the U.S. Food and Drug Administration (FDA) as a source of dietary fibre, but is also thought to have potential therapeutic benefits as an immune stimulating agent and cancer protocol adjunct. Arabinogalactans typically have molecular weights which vary from 10 kDa to 4000 kDa. They typically contain <10% protein, which is normally composed mainly of proline/hydroxyproline, alanine, serine, and threonine. The major part of arabinogalactans (>90%) consist of polysaccharide, composed mainly of β-(1-3)-galactan chains with β-(1-6)-galactosyl side chains terminated primarily with arabinosyl residues.

Green coffee and roasted coffee are known to contain arabinogalactans but the isolation of these arabinogalactans is considered prohibitively difficult and suitable practical procedures to obtain arabinogalactans from coffee are presently unknown. Application of procedures which are known to the person skilled in the art lead, when applied to coffee, to excessive degradation of the arabinogalactan with consequently a strong reduction in molecular weight and significant chemical modifications which render its application as a viscosity modifier and encapsulation matrix useless.

The present enzymatic process surprisingly provides a method for the isolation of arabinogalactan-protein from green and roasted coffee beans, i.e. coffee-derived arabinogalactan, which has controlled molecular weight and which has been minimally degraded.

The process of the present invention refers to the extraction of arabinogalactans from coffee comprising enzymatically hydrolysing whole or ground green or roasted coffee beans thereby obtaining an aqueous dispersion comprising partially hydrolysed coffee beans and arabinogalactans, further, preferably, comprising:
treating the aqueous dispersion to yield an aqueous solution containing arabinogalactans, or optionally a powder enriched in arabinogalactans;
isolating the arabinogalactans by concentrating the aqueous solution and precipitating the arabinogalactan, thereby obtaining a dispersion of extracted arabinogalactans.

We have surprisingly found that arabinogalactans can be obtained from green and roasted coffee using enzymatic treatment. Arabinogalactan is released from green and roasted coffee beans by enzymatic hydrolysis of the mannan-cellulose component of the wall with Gammanase and Celluclast enzyme preparations preferably followed by water-extraction of the powdered beans and subsequently, suitably purified by concentration and precipitation.

The enzymatic hydrolysis of the green and/or roasted coffee beans is preferably carried by the following steps: First, the coffee beans are ground to an average particle size between 10 µm and 2 mm, preferably between 100 µm and 800 µm and more preferably between 200 µm and 400 µm. Next, the ground coffee beans are pre-treated with water at a temperature between 10 °C and 95 °C, preferably between 40 °C and 80 °C and more preferably between 50 °C and 70 °C, for 2 h up to 1 week, preferably for 12 h up to 48 h and more preferably for 20h to 28 h. Afterwards, the pre-treated ground coffee is incubated with cellulase and gamanase. The incubation is carried out at a temperature between 30 °C and 80 °C, preferably between 40 °C and 70 °C and more preferably between 55 °C to 65 °C, for 12 h to 1 week, preferably 48 h to 96 h, more preferably between 50 h to 70 h. After completion of the incubation, the suspension is cooled down to ambient temperatures and the solid residue is removed to yield a soluble fraction.

This soluble fraction contains the extracted arabinogalactans but for many purposes it needs to be further treated. It is obvious that the precise manner in which the step for treating the aqueous dispersion to yield an aqueous solution of arabinogalactans (step a), and the subsequent isolation and concentration step (step b) are carried out may vary depending on the source of the coffee beans, the degree of roasting, and on the required properties for the final application. How to beneficially apply such variations is known to the person skilled in the art, but for illustrative purposes we provide a number of specific embodiments of each of the steps a and b.

First, however, we illustrate how a person skilled in the art may employ different unit operations to carry out a certain generic operation within the framework of the present invention. For instance, the concentration operation which preferably is applied in the present process, can be carried out by any of the techniques known to the person skilled in the art. We have found that concentration carried out by evaporation or ultrafiltration to be particularly useful. Furthermore, if drying processes are used, any drying method may be deployed such as spray-drying, freeze-drying, vacuum-drying, belt drying or fluidized bed drying, or any combination of the mentioned drying methods. Again, the use of such drying technologies is known to the person skilled in the art.

A preferred embodiment of treating the aqueous dispersion to yield an aqueous solution comprising arabinogalactans comprises the following steps: The soluble fraction obtained after the enzymatic hydrolysis is dilute and is concentrated prior to further manipulation. The soluble fraction may be concentrated to between 5 % and 95 %, preferably to between 10% and 80%, more preferably from about 20 % to about 30 % of its original volume. Subsequently, the concentrated soluble fraction is centrifuged in order to separate a small residue and a creamy, brown surface layer. This creamy, brown layer is removed yielding a clear a brown-coloured supernatant containing extracted arabinogalactans. If desired, this supernatant may be dried to yield a powdrous extract enriched in arabinogalactans, but it can also be further manipulated in the liquid state.

The isolation step (step b) of the process may be carried out according to the following steps. First, the supernatant of the clear brown-coloured supernatant is diluted with water rendering a solution. If a powder was previously obtained, this may be dissolved in water to the same concentration. If desired, low molecular weight material may be removed by dialysis of the solution using a membrane with a molecular weight cut-off of 5 kDa to 100 kDa, preferably 10 to 50 kDa, more preferably around 14 kDa. If needed, the dialyzed solution may be concentrated to a viscous liquid. The arabinogalactans in solution are then precipitated by mixing or incubating the solution with 50 to 90 volume %, preferably 60 to 80 volume %, more preferably around 70 volume % of a liquid which is essentially miscible in water. Such a liquid is preferably water-soluble organic solvents, such as low molecular weight alcohols, aldehydes and ketones, or mixtures of the three compounds. Water-soluble alcohols are specifically preferred, exemplified by ethanol, propanol , butanol and isomers thereof. Afterwards, the precipitate may be washed and dried. The final product is an off-white powder having an arabinogalactan content above 50%, preferably above 70%, more preferably above 80%.

According to a further preferred embodiment of the present invention, the arabinogalactans are extracted from green beans only.

The arabinogalactans thus enzymatically extracted from green or roasted coffee can be beneficially used as a food ingredient. They may in particular be used in food to enhance the sensory qualities of the food. The term food also encompasses beverages and any other product digestible by humans. Important applications are, but not limited to, viscosity modulation, the encapsulation of sensitive active ingredients and the stabilization of foams. A particularly preferred food is beverages and particularly soluble coffee, such as pure soluble coffee. Unexpected novel properties of the extracted arabinogalactans include the unusual rheological behaviour including the interfacial behaviour and the ability to form glassy matrices for encapsulation of sensitive active ingredients, like for instance coffee aroma.

After the extraction and isolation, the properties of the arabinogalactan may be precisely tuned for the final application, for example viscosity control to improve the mouthfeel of a coffee beverage, or the use as encapsulation matrix for coffee aroma, by modifying the molecular weight distribution of arabinogalactans by e.g. hydrolysing the arabinogalactan to the desired molecular weight profile or dialysis with membranes.

The composition and properties of the coffee-derived arabinogalactan may be different, depending on the source of the coffee bean, the roasting conditions in case of roasted coffee and, foremost, on the final application.

For instance, for viscosity modulation and foam or interfacial stabilization, generally a higher-molecular weight arabinogalactan is desired. The present method of extraction is singularly suitable to yield such a high molecular weight arabinogalactan, because of its gentle nature. The weight-average molecular weight preferably used for the viscosity modulation are higher than 100 kDa, preferably higher than 500 kDa more preferably higher than 2000 kDa.

If the molecular weight of the arabinogalactan is too high, as could e.g. be the case when the material is used as encapsulation matrix, it could be controllably reduced by any method known to the person skilled in the art, suitably by hydrolysis under mild conditions using diluted acid as described above. Suitable weight-average molecular weights to form glassy matrices for encapsulation are above 10 kDa, preferably above 30 kDa and more preferably above 100 kDa.

In addition, the purity of the isolated arabinogalactan may vary depending on the required properties for a specific application. For instance, as viscosity modulator, the purity of the extracted arabinogalactan may be above 50%, preferably above 75% and more preferably above 90%. For use as encapsulation matrix, the purity may be above 75%, preferably above 90% and more preferably above 95%.

The coffee-derived arabinogalactans from the enzymatic treatment have a molar ratio of galactose to arabinose above about 2:1, preferably above about 2.5:1. As stated, the extracted arabinogalactans are highly useful as food ingredient, in particular to enhance the sensory properties of foodstuffs. The extracted arabinogalactans improve the sensory properties of foodstuffs principally because of two properties: 1) The enhancement of the viscosity of liquids at low concentrations of added arabinogalactan and 2) The ability to form a glassy matrix suitable to entrap sensitive aroma compounds. In specific cases, the two beneficial properties may be simultaneously exploited.

For use as viscosity enhancer of liquid foodstuffs, in particular beverages such as soluble coffee or ready to drink coffee, the extracted arabinogalactans are added to the food product in final concentrations between 0.1% and 10%, preferably between 0.5% and 5%, more preferably between 1% and 2%.

For use as stabilizer of foams in foodstuffs, the extracted arabinogalactans are added to the food product in final concentrations between 0.1% and 10%, preferably between 0.5% and 5%, more preferably between 1% and 2%.

As matrix for the encapsulation of sensitive food ingredients, the extracted arabinogalactans are used in their glassy state. The properties of this glassy state of the arabinogalactans may be modified by changing the extraction conditions of the arabinogalactans, by adding carbohydrates from other sources, in particular disaccharides or by changing the moisture content. The active ingredients are dispersed in the matrix and may be molecularly dispersed or in the form of small liquid or solid inclusions.

The active ingredients may be any food ingredient conveying added value to the final food product. A non-exhaustive list of example include the following: anti-oxidants, flavours, bioactive ingredients, minerals, probiotics. Of interest is the application of the arabinogalactans to encapsulate flavours, which comprise both taste compounds and aroma compounds. An application of particular interest is the application to the encapsulation of coffee aroma for use in soluble coffee.

With coffee aroma is meant the mixture of volatile compounds which provide those odour/flavour sensations experienced by the drinker by stimulating receptor cells in the olfactory epithelium. Aroma compounds enter the nasal cavity either externally by sniffing through the nose (then the odorant molecule is perceived as an odour) or internally by drinking via the retronasal cavity at the back of the mouth and throat (then it is perceived as a flavour). There are many hundreds of compounds in coffee aroma which have been identified as contributing to the aroma of coffee, some of the most important of which are 2,3-butanedione, 2,3-pentanedione, 1-methylpyrrole, furfuryl thiol (FFT), 1H-pyrrole, methanethiol, ethanethiol, pentanethiol, propanal, butanal, ethanal, methylformate, methylacetate, methylfuran, 2-butanone, methanol, ethanol, propanol, pyrazine, furfural, dimethylsulfide, 4-hydroxy-2,5-dimethyl-3(2*H*)-furanone, 2-methylbutanal, 2(5)-ethyl-4-hydroxyl-5(2)-methyl-3(2H)- furanone, methylpropanal, 4-ethenyl-2-methoxyphenol, 3-methylbutanal, vanillin, 2-methoxyphenol, 3-hydroxy-4,5-dimethyl-2(5*H*)-furanone, 4-ethyl-2-methoxyphenol, 2-ethyl-3,5-dimethylpyrazine, methional, 3-mercapto-3-methylbutylformate, 2,3-diethyl-5-methylpyrazine, (*E*)-□-damascenone, 3-isobutyl-2-methoxypyrazine, 2-methyl-3-furanthiol, 2-ethenyl-3,5-dimethylpyrazine, 3-methyl-2-butene-1-thiol and 2-ethenyl-3-ethyl-5-methylpyrazine.

Coffee aroma as used in the invention denotes any mixture of aroma compounds found in the aroma of coffee, but the coffee aroma need not be of natural origin. For instance, a natural coffee aroma extract or condensate can be enriched by adding certain amounts of defined aroma compounds. These added aroma compounds may be natural, for instance from non-coffee sources, or they may be nature identical. Such a coffee aroma enriched in certain aroma compounds will be denoted as a coffee aroma composition. The same nomenclature we apply to a coffee aroma which is prepared from single, pure aroma compounds or non-coffee aromas. Coffee aroma may be processed as the essentially pure composition containing only the aroma compounds, i.e. concentrate, but it may be also in the form of an extract or condensate containing an aroma carrier, e.g. coffee oil or water, and optionally non-volatile coffee compounds. The coffee aroma in such a carrier will also be denoted as a coffee aroma composition. The concentration of coffee aroma compounds in a coffee aroma composition may vary, depending on its source, its application and on the type of carrier used. For instance, if water is used as a carrier, the concentration of coffee aroma compounds is usually fairly low, for example between 0.001 % and 10%, often between 0.1 % and 1%. Oil-based aromas generally have an aroma concentration between 1% and 90%, preferably between 5% and 20%. Any aroma composition consisting for 90% or more of coffee aroma compounds will be denoted as pure coffee aroma or as coffee aroma concentrate. The coffee aroma according to the present invention may be obtained through any means known to a person skilled in the art.

The encapsulation or entrapment of the coffee aroma in the arabinogalactan matrix may be carried out following any of the techniques commonly used in the art. Such techniques include, but are not limited to spray-drying, freeze-drying, melt extrusion, fluidised-bed drying, spray-drying combined with agglomeration, vacuum drying, or any combination of said encapsulation methods. A general outline of the common techniques can for instance be found in J. Ubbink and A. Schoonman, 'Flavor Delivery Systems', Kirk-Othmer Encyclopedia of Chemical Technology, Wiley Interscience (2003).

The selection of the most appropriate technology is usually determined by optimally satisfying numerous demands on processing, powder properties and consumer preferences. For instance, the choice of technology can be determined by the availability of equipment, its operating costs, the energy input required per unit product and similar considerations. When powder properties are of importance, this choice may be influenced by constraints on powder flowability, reconstitution behaviour and mixing behaviour. Consumer preference may play an important role in the selection of the technology in that powder appearance will influence the perception of the product by the consumer. Solely as an illustrative example, consider the case where the arabinogalactan capsules containing encapsulated coffee aroma are to be use to fortify a spray-dried soluble coffee. Both from the processing perspective, the powder properties as well as the visual acceptance of the product by the consumer it makes sense to use spray-drying as drying technology. In another case, mentioned only because of illustrative purposes, freeze-drying is the technology of choice because the arabinogalactan capsules will be blended with a freeze-dried soluble coffee powder in order to optimise the appearance of the powder mixture. In another illustrative case, the arabinogalactan capsules are produced by fluidised-bed drying and mixed with a freeze-dried soluble coffee powder providing a final powder mixture with a visually pleasing contrast between the freeze-dried coffee particles and the fluidised-bed dried arabinogalactan capsules. However, arabinogalactans-encapsulated coffee aroma obtained by any encapsulating method may be used in conjunction with any powdered soluble coffee.

The process of incorporating the coffee aroma in arabinogalactan capsules, which could also be referred to as entrapment or encapsulation, may be accomplished by any known methods. Suitably, the process comprises the following steps:
- Dissolution of the arabinogalactan in water;
- Addition of the coffee aroma in any physical form as mentioned above;
- Homogenisation of the mixture yielding a homogeneous solution or dispersion;
- Drying of the solution of the dispersion containing the coffee aroma to yield arabinogalactan capsules containing encapsulated coffee aroma.
- Post-processing of the arabinogalactan capsules in order to modify particle size, particle morphology or powder properties.

These steps provide a general description of the process of encapsulation of the coffee aroma extract, concentrate, extract or composition in arabinogalactan capsules. Needless to say, each step may be carried out in various way still encompassed by the above operation, also, the above steps may be deployed in any order or be fully left out. For illustrative purposes, we give a few examples embodiments of the process for incorporating the coffee aroma in arabinogalactan. If, for example, the isolated and purified arabinogalactan is already in the form of an aqueous solution, there is no need not dissolve it in water prior to its use in the encapsulation process. It could even be the case that, if the arabinogalactan solution is highly dilute, we need to concentrate the arabinogalactan solution to the appropriate solids content. Another example is if the coffee aroma is provided as a water-based composition, then one may dissolve the arabinogalactan in the coffee aroma composition without perhaps even adding water. Further, the drying step may include a freezing step if freeze-drying is utilized as drying process. Also, the post-processing may be more or less extensive as for instance after freeze-drying, the resulting cake needing to be milled or broken in order to obtain capsules of the desired sizes. Grinding of the arabinogalactan capsules is often also desired if melt-extrusion is used as encapsulation process. Little or no post-processing is usually required if spray-drying or fluidised-bed drying are employed, as the capsules generally have already the desired particle size and shape. An exception is in case agglomeration is used as post-processing step. Agglomeration may be used to increase the particle size, to improve the powder properties or to modify the visual appearance of the capsules. Agglomeration may be carried out using the capsules, or using the capsules in combination with the coffee powder, or, equivalently, any of the other ingredients of a beverage powder, such as creamer and sweetener.

By the above outlined process for encapsulating the coffee aroma, particles are formed predominantly comprising coffee-derived arabinogalactans by weight, typically above about 70 %, preferably above about 80 % by weight. Said obtained particles, which can be referred to as a glassy matrix comprising coffee-derived arabinogalactans, are suitably mixed with a soluble coffee powder thereby rendering a soluble coffee composition, such as a pure soluble coffee composition, comprising coffee-aroma encapsulated in coffee-derived arabinogalactans. The coffee-derived arabinogalactans have suitably a weight-average molecular weight above about 10 kDa, preferably above about 30 kDa, furthermore, the molar ratio of galactose to arabinose is typically above about 2:1, preferably above about 2.5:1. The soluble coffee composition comprising coffee-aroma encapsulated in coffee-derived arabinogalactans has above about 10 weight % of coffee-derived arabinogalactans from both the novel enzymatic extraction procedure and the traditional extraction based on total soluble coffee composition. Here, soluble coffee composition is meant only those compounds originating from coffee beans, i.e. green beans and roasted beans, thus, not additives such as creamer, sweetener or other common additives. To the so formed soluble coffee composition, other constituents are preferably admixed, thereby obtaining various coffee beverages comprising creamer and optionally sweetener.

### Example 1: Extraction of arabinogalactans from green coffee

Celluclast 1.5 L and Gamanase were purchased from Novo-Nordisk. Celluclast 1.5 L is a liquid cellulase preparation from *Trichoderma* reesi. The activity of this preparation is 700 *endo-*glucanase units /g. Gamanase is obtained from an *Aspergillus* organism and has an activity of 1,000,000 viscosity enzyme units per g. Each enzyme was partially purified by adding 200 ml of the enzyme preparation to 800 ml of 95% alcohol at 4°C. The protein precipitate which formed was recovered after a wash in 80% alcohol and redissolved in 200 ml of water just before use. Green coffee beans were milled to an average particle size between 200-400 µm. Liquid nitrogen was used as a coolant. 10 kg of powdered beans were stirred for 24 h at 60°C in distilled water. The enzymes (200 ml of the dissolved precipitates) were added to the bean suspension and stirring continued for 62 h at 60°C. The mixture was allowed to cool and the residue separated from the soluble fraction. The soluble fraction was then concentrated to a quarter of its original volume and the concentrate centrifuged at 7000 rpm in 1 L containers using a laboratory centrifuge. A fatty, brown, creamy layer formed on the surface and was removed and the clear, brown, coloured supernatant was freeze-dried yielding a powder containing coffee-derived arabinogalactans. The presence of arabinogalactans was detected by size-exclusion chromatography, the weight-average molecular weight was determined to be 3.78 x10⁶ Da, the number-average molecular weight was Mn = 2.83 x 106, giving a Mw/Mn ratio of 1.34. The molar ratio of galactose to arabinose was determined to be 3:1, as determined from the arabinose content of 22.6 mole % and the galactose content of mole 68.4 %.

### Example 2: Extraction of arabinogalactans from roasted coffee

The procedure of Example 1 was applied, but now on roasted coffee (CTN 110). The presence of the arabinogalactans was detected by size-exclusion chromatography, the weight-average molecular weight were determined to be in the range between 10 and 100 kDa. The arabinose content of the extract was 26.3 mole %, the galactose content was determined to be 67.3 mole %, giving a molar ratio of galactose to arabinose of about 2.5.

### Example 3: Isolation of arabinogalactans from the extract and preparation of an arabinogalactan powder

The freeze-dried extract of Example 1 (500 g) was dissolved in 700 ml water and dialysed in a membrane with molecular weight cut-off of 14 kDa. The solution was concentrated to a viscous liquid (-1300 ml) and alcohol (95%) added slowly with constant stirring until it reached a final concentration of 70% (3000 ml). The precipitate was allowed to settle overnight at ambient temperature and most of the supernatant decanted. The precipitate was re-suspended in 70% alcohol, centrifuged and the supernatant discarded. The precipitate was resuspended and stirred in acetone (200 ml), centrifuged and the supernatant discarded. Finally the precipitate was suspended in a little acetone and poured into crystallising dishes where it was allowed to dry in a fume hood overnight at room temperature. The arabinogalactan was recovered as a white material which was crushed to a powder (yield 80 g). The arabinogalactans content was 85 wt. % and the protein content was 10 wt. %. The galactose/arabinose ratio was 3:1 and it contained also about 7 wt. % glucuronic acid.

### Example 4: Use of coffee-derived arabinogalactans to deliver enhanced texture in beverages

Soluble coffee beverages were prepared using 2 gram of Nescafe Gold soluble coffee per 150 ml of demineralized water. The temperature of the water was 70 °C. Samples were prepared without arabinogalactan, and with 1% and 2% w/w arabinogalactan of Example 3. The samples were judged by a panel of 5 tasters who were asked to qualitatively assess the differences between the three samples. Common opinion was that the body of the beverages containing the 1% and 2% arabinogalactan was more substantial, with some film-forming in the mouth. In addition, two tasters judged the arabinogalactan-containing beverage to have higher foam-forming ability.

In parallel, the rheological behaviour of 1% and 2% solutions of arabinogalactan of Example 3 was determined using a Haake RS150 RheoStress rheometer. The experiments were carried out at 37 °C using a so-called double-gap setup. This setup consists of a hollow stainless-steel cylinder suspended in a concentric manner between two other stainless steel cylinders.

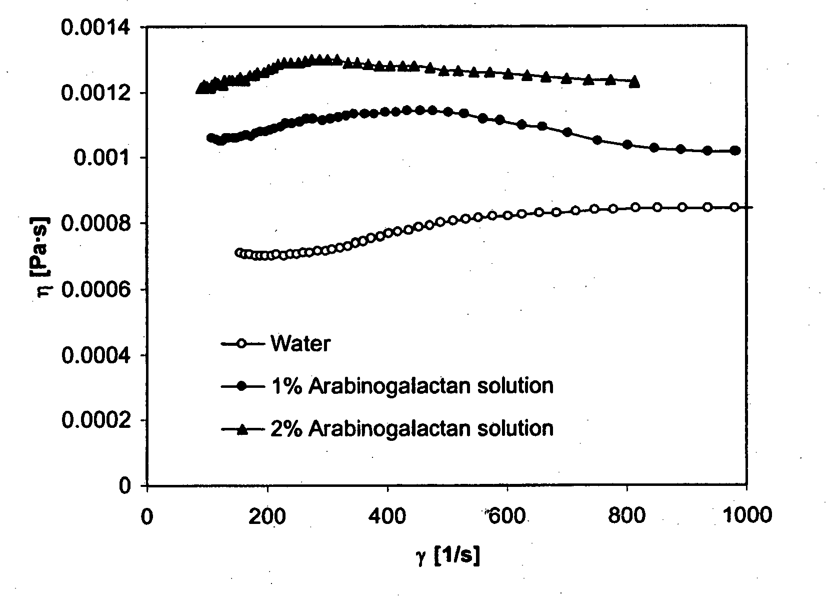

From the rheological data it is observed that at arabinogalactan concentrations of 1% and 2%, the viscosity of the solution is already substantially higher than of water. As during consumption of a food product the shear rate may vary between about 1 1/s and 300 1/s, a relevant quantitative measure of the viscosity is the viscosity at 200 1/s:

| **Sample** | **Viscosity at a shear rate of 200 1/s; 37 °C [Pa.s]** |
|---|---|
| 1 % Arabinogalactan in water | 0.0011 |
| 2% Arabinogalactan in water | 0.0013 |
| water | 0.00070 |

From the value of the viscosity at a shear rate of 200 1/s, we indeed infer that the arabinogalactan has a strong tendency to increase the viscosity of the solution.

From both the sensory evaluation and the rheological measurements we conclude that the coffee-derived arabinogalactan is a suitable compound to modify the texture of a beverage.

### Example 5: Foaming properties

Acacia gum and coffee arabinogalactan powders were dispersed at a concentration of 5 wt% in deionised MilliQ water (18.2 M□) to test the foaming properties of the hydrocolloids in non-buffered conditions. Determination of the foaming properties of aqueous dispersions is dependent on the technique and apparatus used. To avoid these problems, we have chosen to use a standardized foaming method developed by Guillerme et al. (Journal of Texture Studies, 24, 287-302 (1993)). The principle is to foam a defined quantity of solution by gas sparkling through a glass frit (porosity and gas flow are controlled). The generated foam rises along a glass column where its volume is followed by image analysis using a CCD camera. The amount of liquid incorporated in the foam and the foam homogeneity are followed by measuring the conductivity in the cuvette containing the liquid and at different heights in the column by means of electrodes. The commercial Foamscan apparatus (ITConcept, Longessaigne, France) has been used to perform these experiments. The foaming capacity of coffee arabinogalactan and acacia gum were compared at various pH values.

| *Comparative foaming properties of coffee arabinogalactan and Acacia gum solutions* | | |
|---|---|---|
| | Coffee AG | Acacia gum |
| *Foam capacity (5%, pH 5.5) | 1.0 | 0.82 |
| **Foam stability (5%, pH 5.5) | 60 | 60 |
| ***Surface tension y (0.5% solution in water) | 50 | 64 |

| | | |
|---|---|---|
| *Volume of foam/volume of gas | | |
| ** Values are % of original foam volume remaining after 30 min | | |

| | | |
|---|---|---|
| *** Equilibrium values in N.m⁻¹ (thus the coffee AG was more surface active than acacia gum) | | |

The foam capacity was higher for the coffee arabinogalactan, compared to acacia gum. The higher foaming capacity of the coffee-derived AG is confirmed by the surface tension, which, at the same concentration by weight, is lower for the coffee-derived arabinogalactan. We conclude that coffee-derived arabinogalactan has improved foam capacity at unchanged stability. Hence, coffee-derived arabinogalactan is an excellent foaming ingredient for application in food and beverages.

### Example 6: Encapsulation of coffee aroma in coffee-derived arabinogalactans

For the encapsulation trials, an aqueous coffee aroma extract was used. The arabinogalactan powder of Example 3 was dissolved at room temperature in fresh aqueous coffee aroma extract (15x the stoichiometric coffee aroma) up to a total solid content of the concentrate of 25.9%. Because an aqueous extract was used, no water needed to be added. After complete dissolution of the arabinogalactan powder and after homogenisation, the resulting coffee extract was put in the freezer at -80°C and then freeze-dried under controlled conditions.

After freeze-drying, a porous, glassy matrix was obtained containing encapsulated coffee aroma. The glassy matrix could easy be crunched yielding a free flowing powder with an apparent density of 1.64 g/cm³ as determined by gas pycnometry.

The retention of the aroma in the arabinogalactan capsules was determined using GC analysis. For a number of the impact compounds from coffee aroma, the results are summarized below:

| Compound | Retention after freeze drying [%] |
|---|---|
| acetaldehyde | 89.5 |
| methyl formate | 57.6 |
| propanal | 70.4 |
| methyl acetate | 76.9 |
| methylfuran | 72.6 |
| 2-butanone | 70.6 |
| methanol | 51.3 |
| butanal | 65.5 |
| ethanol | 88.0 |
| 2,3-butanedione | 58.5 |
| 2,3-pentanedione | 66.1 |
| propanol | 55.8 |
| pyrazine | 69.9 |
| furfural | 57.5 |
| pyrrole | 62.3 |

As can be seen from this table, aroma retention is satisfactory as all compounds, including the highly volatile ones, are well retained in the matrix.

### Example 7: Storage test to determine the stability of coffee aroma in arabinogalactan capsules

Arabinogalactan capsules were produced according to Example 6 with a concentration of coffee aroma which was 15 times higher than in standard soluble coffee. As a reference, freeze-dried coffee was prepared following standard practice, but with an aroma concentration 15 times higher than normal. Both the arabinogalactan capsules and the soluble coffee reference were equilibrated at a_{w} = 0.32 by storage at 25 °C in desiccators containing a saturated salt solution (M_{g}Cl₂). The water activity was measured using a relative humidity sensor (Hygrolyt, Rotronic AG, Switzerland). After equilibration, a storage test was conducted over a three month period at two temperatures, -25°C and +37°C, on both the arabinogalactan capsules and the soluble coffee reference. At regular intervals, the concentration of several aroma compounds in the capsules and reference stored at the two temperatures was determined. The stability of the aroma compounds is expressed as the relative retention which is calculated as the ratio of the concentration of an aroma compound in a sample stored at 37 °C versus the concentration of the aroma compound in the same sample, but stored at -25 °C. The relative retention turned out to be higher for the arabinogalactan capsules, but the improvement in stability between the arabinogalactan capsules and the soluble coffee reference varied from compound to compound. This is illustrated in the graphs below, where for acetaldehyde and pyrrole a major improvement is observed for the arabinogalactan capsules in comparison with the soluble coffee reference.

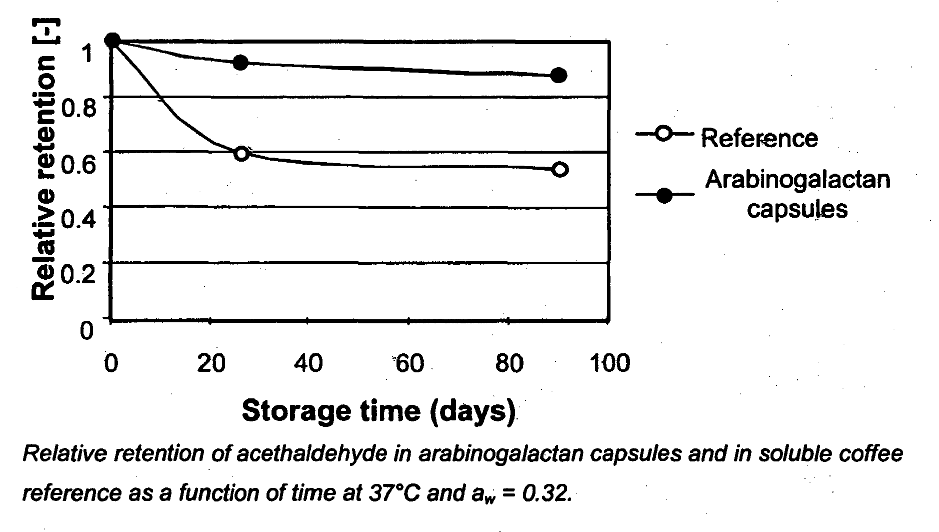

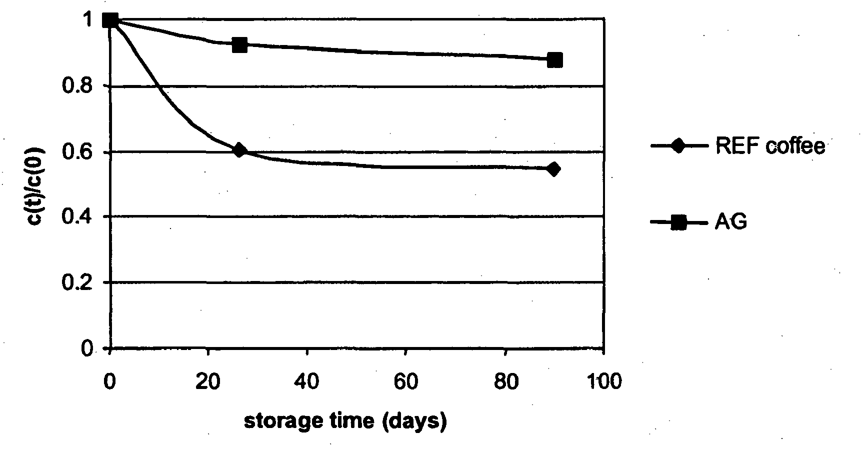

### Example 8: Coffee mixes containing coffee aroma - arabinogalactan capsules

Arabinogalactan capsules were produced according to Example 6 with a concentration of coffee aroma which was 15 times higher than in standard soluble coffee. The arabinogalactan capsules were mixed with non-aromatised soluble coffee prepared by freeze-drying in a weight ratio of 1 to 15 yielding a coffee mix with a standard overall aroma concentration. The coffee mix obtained in this way had a pleasant visual appearance. In addition, the tendency of the arabinogalactan capsules and the soluble coffee to separate in the powder mix acceptable.

### Example 9: Improved sensory characteristics

Arabinogalactan capsules were produced according to Example 6 with a concentration of coffee aroma which was 15 times higher than in standard soluble coffee. As a reference, freeze-dried coffee was prepared following standard practice, but with an aroma concentration 15 times higher than normal. The production of the boosted samples was kept identical with respect to the amount of aroma added, the total solids content before freeze-drying and the freeze-drying equipment and conditions. Sensory profiles of the samples were carried out immediately after production of the samples (T₀). Samples for tasting were made up as follows: Temperature of water: 70°C. Type of water: 2/3 mineral water and 1/3 deionised water. The coffee powders were diluted according to concentrations mentioned in the Table below. The total amount of boosted material in finished product is about 6-7%.

| *Coffee powders used for tasting.* | | |
|---|---|---|
| Sample code | Raw material | Concentrations used for tasting (%) |
| 1 | Coffee A (standard coffee matrix - reference) | 1.5 |
| 2* | Coffee A boosted with coffee aroma (15x stoichiometry; reference). | 0.1 |
| 3* | Arabinogalactan from green coffee boosted with coffee aroma (15x stoichiometry) | 0.1 |

| | | |
|---|---|---|
| * Note that products 2 and 3 were completed with Coffee A with no aroma reincorporated to have the same 1.5 % final coffee concentration. | | |

Coffee beverages are evaluated by a panel of 11 trained tasters, using 20 odour/flavour attributes. Assessors were asked to score each attribute on an 11-point scale ranging from 0 (not intense) to 10 (very intense).

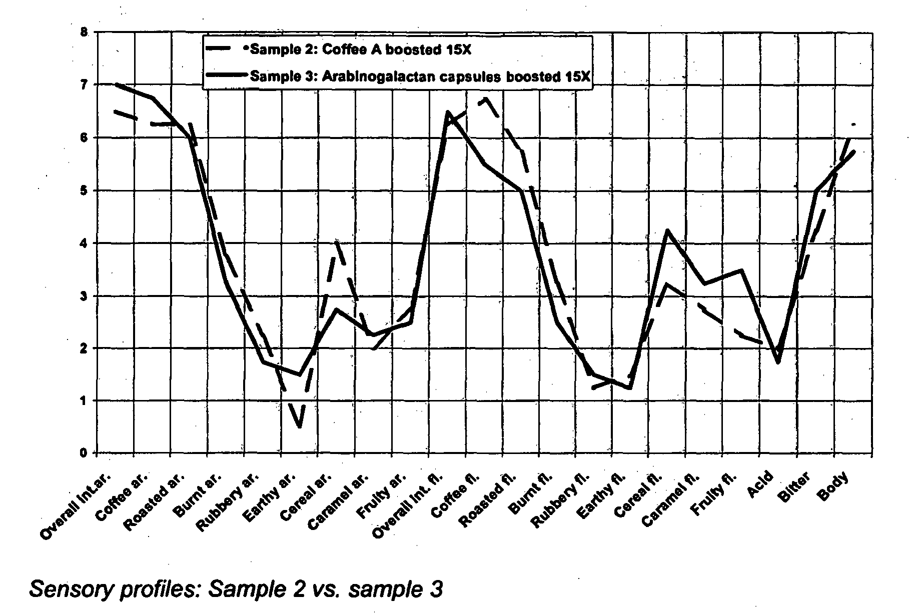

These three products were found to have a high coffee character in aroma and flavour. The use of an arabinogalactan matrix slightly increases the cereal, caramel and fruity notes of sample 3 as compared to sample 2, whereas coffee and roasty flavour attributes are slightly weaker. Overall, these differences were not significant. Hence, we conclude that the arabinogalactan capsules do not significantly change ? the sensory impact of the coffee to which they are added at the beginning of the storage. Soluble coffee can therefore be boosted with arabinogalactan capsules without significantly changing the initial sensory character of the soluble coffee.

A storage test was performed to assess the relative stability of the aroma in the arabinogalactan capsules and in the boosted soluble coffee. Both the arabinogalactan capsules and the soluble coffee reference were equilibrated at a_{w} = 0.32 by storage at 25 °C in dessicators containing a saturated salt solution (M_{g}Cl₂). After equilibration, the samples were stored at two temperatures, -25°C and +37°C, for a three month period. The coffee base powder with no aroma reincorporated was stored at -25°C and 37°C but at low moisture content (a_{w} = 0.17) to prevent degradation of non-volatiles and acidity development.

Triangle tests were conducted between samples of each product kept at -25°C and +37°C after 1 month storage (T₁) and after the full 3 months storage period (T₃). For tasting the beverages were reconstituted with 1.4g non-aromatised soluble coffee powder and 0.1 g boosted powder (arabinogalactan capsules and boosted soluble coffee powder) per 100 mL cup.

| *Results of the triangle test for each product at 1 month and 3 months. The asteriks indicates that the products are significantly different (P < 0.05).* | | | | |
|---|---|---|---|---|
| **Comparison -25 °C vs. +37 °C** | **1 month storage (T₁)** | | **3 month storage (T₃)** | |
| *Sample* | *Correct* | *Significance* | *Correct* | *Significance* |
| reference | 13/20 | 0.04* | 12/20 | 0.013* |
| Arabinogalactan capsules | 9/20 | NS | 6/20 | NS |

The data in the Table above is a measure of the effect of storage for 1 month and 3 months on the odour/flavour attributes of various coffee aroma compositions (left-hand column). The figures (X/Y) represent the number of assessors who evaluated the batches stored at -25°C and +37°C as different (X) over the total number of tests (Y). Hence, a high ratio of X to Y signifies that the coffee aroma composition was judged to be significantly different in odour/flavour after storage for 1 or 3 months.

The results from the triangle test show that, whereas for the soluble coffee reference, differences between the sample stored at -25 °C and +37 °C are significant already after one month of storage under severe conditions (T=37°C, a_{w} 0.32), no significant variations are observed for the arabinogalactan capsules even after three months of storage under these severe conditions. We conclude that the entrapment of coffee aroma in arabinogalactan capsules is beneficial for preserving the initial aroma quality and strength of soluble coffee.

## Claims

1. A process for extracting arabinogalactans from coffee comprising enzymatically hydrolysing whole or ground green or roasted coffee beans thereby obtaining an aqueous dispersion comprising partially hydrolysed coffee beans and arabinogalactans; the process further comprising the steps:
a) treating the aqueous dispersion to yield an aqueous solution comprising arabinogalactans,
b) isolating the arabinogalactans by concentrating the aqueous solution and precipitating the arabinogalactan, thereby obtaining a dispersion of extracted arabinogalactans.

2. The process according to claims 1, further comprising one or more of the following steps:
c) hydrolysing of the extracted arabinogalactans of claim 1 to reduce the molecular weight;
d) concentrating the aqueous dispersion to obtain a viscous liquid with a high concentration of coffee-derived arabinogalactans;
e) drying the arabinogalactan precipitate of step b) or the viscous liquid of step d) to obtain a solid preparation of coffee-derived arabinogalactans.

3. The process according to claim 1, wherein the enzymatic hydrolysis comprises the following steps:
- grinding of the coffee beans to an average particle size between about 10 micrometer up to about 2 millimeter;
- pre-treating of the ground coffee beans with water at a temperature from about 10 °C up to about 95 °C for about 2 hours up to about 1 week;
- incubating the pre-treated ground coffee at a temperature of from about 30 °C up to about 80 °C with cellulase and gamanase for about 12 hours up to about 1 week;
- cooling of the suspension of step c) to ambient temperatures;
- removing the residue of the suspension of step. c to yield a soluble fraction.

4. The process according to claim1, wherein step a) comprises the additional steps:
- concentrating the aqueous solution comprising arabinogalactans to between 5 % and 95 % of its original volume;
- centrifuging the concentrated aqueous solution thereby obtaining a surface layer;
- removing the surface layer of the centrifuged solution yielding a supernatant containing extracted arabinogalactans.;
- optionally, drying the supernatant of step b) to yield a powdrous extract enriched in arabinogalactans.

5. The process according to claim 1, wherein step b) comprises the additional steps:
- diluting the supernatant or dissolving the dried extract enriched in arabinogalactans in water giving an aqueous solution;
- optionally dialysing the aqueous solution using a membrane with a molecular weight cut-off of about 5 kDa to about 100 kDa;
- concentrating of the aqueous solution or the optionally dialyzed solution to a viscous liquid;
- incubating the viscous liquid with about 50 to about 90 volume % of water-soluble organic solvents to obtain a precipitate.
- optionally washing the precipitate with aqueous alcohol solutions and acetone.
- optional drying the precipitate.

6. The process according to any of the claims 1, 2, 4 and 5, wherein the concentration is carried out by evaporation or ultrafiltration.

7. The process according to any of the claims 2, 4 and 6, wherein the drying is carried out using spray drying, freeze drying, vacuum drying, belt drying or fluidised-bed drying.

8. The process according to any of the preceding claims, **characterised in that** arabinogalactans are enzymatically extracted from whole or ground green coffee beans.

9. The use of coffee-derived arabinogalactans obtained by the process of any of the claims 1-8 as a food ingredient.

10. The use of coffee-derived arabinogalactans obtained by the process of any of the claims 1-8 having a weight average molecular weight above about 150 kDa, preferably above about 500 kDa, and more preferably above about 2000 kDa as a texturiser in food.

11. The use of coffee-derived arabinogalactans obtained by the process of any of the claims 1-8 as a viscosity enhancer in food and beverages.

12. The use of coffee-derived arabinogalactans obtained by the process of any of the claims 1-8 as a mouthfeel enhancer for soluble coffee.

13. The use of coffee-derived arabinogalactans obtained by the process of any of the claims 1-8 as a texturiser in beverages.

14. The use of coffee-derived arabinogalactans obtained by the process of any of the claims 1-8 as a foam enhancer in food and beverages.

15. The use of coffee-derived arabinogalactans obtained by the process of any of the claims 1-8 as a foam stabilizer in coffee beverages.

16. The use of coffee-derived arabinogalactans obtained by the process of any of the claims 1-8 as a glassy matrix in dehydrated food.

17. The use of coffee-derived arabinogalactans obtained by the process of any of the claims 1-8 as a matrix to entrap flavours or aromas, or mixtures thereof in dehydrated food.

18. The use of coffee-derived arabinogalactans obtained by the process of any of the claims 1-8 as a matrix according to claim 18, **characterised in that** the dehydrated food is a beverage.

19. The use of coffee-derived arabinogalactans as a matrix to entrap coffee aroma.

20. A solid coffee composition comprising coffee aroma entrapped in the coffee-derived arabinogalactans of which the aroma is perceivably stronger and of preferred quality after prolonged storage as compared to standard soluble coffee.

21. A glassy matrix comprising coffee aroma, **characterised in that** the matrix comprises coffee-derived arabinogalactans.

22. The glassy matrix according to claim 21, **characterised in that** the coffee-derived arabinogalactans have an average molecular weight above about 10 kDa.

23. The glassy matrix according to claim 21, **characterised in that** the average molecular weight is above about 30 kDa.

24. The glassy matrix according to claim 21, wherein the matrix comprises above about 80 weight % of coffee-derived arabinogalactans based on total solid glassy matrix.

25. The glassy matrix according to any of claims 21 to 24, **characterised in that** the coffee-derived arabinogalactans have a weight ratio of galactose to arabinose above about 2:1.

26. The glassy matrix according to claim 25, **characterised in that** the weight ratio of galactose to arabinose above about 2.5:1.

27. The glassy matrix according to any of claims 21 to 25, **characterised in that** the matrix is obtainable by mixing a coffee aroma with an aqueous dispersion of coffee-derived arabinogalactans thereby forming a mixture, and thereafter processing the obtained mixture to form the solid glassy matrix.

28. The glassy matrix according to claim 27, **characterised in that** the processing comprises spray-drying, spray-drying and agglomeration, freeze-drying, melt extrusion, or vacuum drying, or a combination thereof.

29. A pure soluble coffee composition comprising the glassy matrix as defined by claim 21.

30. A soluble coffee beverage composition according to claim 29, **characterised in that** it further comprises a creamer and optionally a sweetener.

## Patentansprüche

1. Verfahren zur Extraktion von Arabinogalaktanen aus Kaffee bestehend aus der enzymatischen Hydrolyse ganzer oder gemahlener Rohkaffe- oder Röstkaffeebohnen, so dass man eine wässrige Dispersion mit teilweise hydrolysierten Kaffeebohnen und Arabinogalaktanen erhält, wobei das Verfahren die nachfolgenden Schritte aufweist:
a) Aufbereiten der wässrigen Dispersion, um eine wässrige Lösung mit Arabinogalaktanen zu erhalten,
b) Isolieren der Arabinogalaktane durch Aufkonzentrieren der wässrigen Lösung und Ausfällen des Arabinogalaktan, so dass man ein Dispersion extrahierter Arabinogalaktane erhält.

2. Verfahren nach Anspruch 1, das weiterhin einen oder mehrere der nachfolgenden Schritte aufweist:
c) Hydrolysieren der extrahierten Arabinogalaktane aus Anspruch 1, um das Molekulargewicht zu reduzieren;
d) Aufkonzentrieren der wässrigen Dispersion, um eine viskose Flüssigkeit mit einer hohen Konzentration aus Kaffee gewonnener Arabinogalaktane zu erhalten;
e) Trocknen des Arabinogalaktan-Präzipitats aus Schritt b) oder der viskosen Flüssigkeit aus Schritt d), um ein festes Produkt aus, aus Kaffee gewonnener, Arabinogalaktanen zu erhalten.

3. Verfahren nach Anspruch 1, wobei die enzymatische Hydrolyse die folgenden Schritte aufweist:
- Vermahlen der Kaffeebohnen auf eine durchschnittliche Partikelgröße zwischen ca. 10 Mikrometer bis ca. 2 Millimeter;
- Vorbehandeln der gemahlenen Kaffeebohnen mit Wasser bei einer Temperatur von ca. 10 °C bis ca. 95 °C über ca. 2 Stunden bis ca. 1 Woche;
- Inkubieren des vorbehandelten, gemahlenen Kaffees bei einer Temperatur von ca. 30 °C bis ca. 80 °C mit Cellulase und Gamanase über ca. 12 Stunden bis ca. 1 Woche;
- Abkühlen der Suspension aus Schritt c) auf Umgebungstemperaturen;
- Entfernen des Rückstandes der Suspension aus Schritt c), so dass man eine lösliche Fraktion erhält.

4. Verfahren nach Anspruch 1, wobei Schritt a) die folgenden weiteren Schritte aufweist:
- Aufkonzentrieren der wässrigen Lösung mit Arabinogalaktanen auf 5 % bis 95
% ihres ursprünglichen Volumens;
- Zentrifugieren der konzentrierten wässrigen Lösung, so dass man eine Deckschicht erhält;
- Entfernen der Deckschicht der zentrifugierten Lösung, so dass man einen Überstand mit extrahierten Arabinogalaktanen erhält;
- wahlweise: Trocknen des Überstandes aus Schritt b), so dass man einen mit Arabinogalaktanen angereicherten, pulverförmigen Extrakt erhält.

5. Verfahren nach Anspruch 1, wobei Schritt b) die folgenden weiteren Schritte aufweist:
- Verdünnen des Überstandes mit oder Auflösen des getrockneten, mit Arabinogalaktanen angereicherten Extraktes in Wasser, so dass man eine wässrigen Lösung erhält;
- wahlweises Dialysieren der wässrigen Lösung mittels einer Membran mit einer Ausschlussgrenze von ca. 5 kDa bis ca. 100 kDa;
- Aufkonzentrieren der wässrigen Lösung oder der optional dialysierten Lösung auf eine viskose Flüssigkeit;
- Inkubieren der viskosen Flüssigkeit mit ca. 50 bis ca. 90 Volumen-% wasserlöslicher, organischer Lösungsmittel, um ein Präzipitat zu erhalten;
- wahlweises Waschen des Präzipitats mit wässrigen Alkohollösungen und Azeton;
- wahlweises Trocknen des Präzipitats.

6. Verfahren nach einem der Ansprüche 1, 2, 4 und 5, wobei das Aufkonzentrieren durch Verdampfung oder Ultrafiltration durchgeführt wird.

7. Verfahren nach einem der Ansprüche 2, 4 und 6, wobei das Trocknen mittels Sprühtrocknung, Gefriertrocknung, Vakuumtrocknung, Bandtrocknung oder Wirbelschichttrocknung durchgeführt wird.

8. Verfahren nach einem der vorhergehenden Ansprüche, **dadurch gekennzeichnet, dass** die Arabinogalaktane aus ganzen oder gemahlenen Rohkaffeebohnen enzymatisch extrahiert werden.

9. Verwendung von aus Kaffee gewonnenen Arabinogalaktanen, die man durch das Verfahren nach einem der Ansprüche 1-8 erhalten hat, als Lebensmittelzutat.

10. Verwendung von aus Kaffee gewonnenen Arabinogalaktanen, die man durch das Verfahren nach einem der Ansprüche 1-8 erhalten hat, und die ein gewichtsdurchschnittliches Molekulargewicht von ca. 150 kDa, vorzugsweise über ca. 500 kDa, und bevorzugter über ca. 2000 kDa besitzen, als Texturgeber in Lebensmitteln.

11. Verwendung von aus Kaffee gewonnenen Arabinogalaktanen, die man durch das Verfahren nach einem der Ansprüche 1-8 erhalten hat, als Viskositätsverbesserer in Lebensmitteln und Getränken.

12. Verwendung von aus Kaffee gewonnenen Arabinogalaktanen, die man durch das Verfahren nach einem der Ansprüche 1-8 erhalten hat, um das Mundgefühl von löslichem Kaffee zu verbessern.

13. Verwendung von aus Kaffee gewonnenen Arabinogalaktanen, die man durch das Verfahren nach einem der Ansprüche 1-8 erhalten hat, als Texturgeber in Getränken.

14. Verwendung von aus Kaffee gewonnenen Arabinogalaktanen, die man durch das Verfahren nach einem der Ansprüche 1-8 erhalten hat, als Schaumverbesserer in Lebensmitteln und Getränken.

15. Verwendung von aus Kaffee gewonnenen Arabinogalaktanen, die man durch das Verfahren nach einem der Ansprüche 1-8 erhalten hat, als Schaumstabilisator in Kaffeegetränken.

16. Verwendung von aus Kaffee gewonnenen Arabinogalaktanen, die man durch das Verfahren nach einem der Ansprüche 1-8 erhalten hat, als Glasmatrix in dehydrierten Lebensmitteln.

17. Verwendung von aus Kaffee gewonnenen Arabinogalaktanen, die man durch das Verfahren nach einem der Ansprüche 1-8 erhalten hat, als Matrix zum Einfangen von Geschmack oder Aromen, oder Mischungen davon, in dehydrierten Lebensmitteln.

18. Verwendung von aus Kaffee gewonnenen Arabinogalaktanen, die man durch das Verfahren nach einem der Ansprüche 1-8 erhalten hat, als Matrix nach Anspruch 18, **dadurch gekennzeichnet, dass** es sich bei dem dehydrierten Lebensmittel um ein Getränk handelt.

19. Verwendung von aus Kaffee gewonnenen Arabinogalaktanen, die man durch das Verfahren nach einem der Ansprüche 1-8 erhalten hat, als Matrix zum Einfangen von Kaffeearoma.

20. Feste Kaffeezusammensetzung mit Kaffeearoma, das in den aus Kaffee gewonnenen Arabinogalaktanen eingeschlossen ist, bei der das Aroma im Vergleich zu normalem löslichen Kaffee nach längerer Lagerung wahrnehmbar stärker und von bevorzugter Qualität ist.

21. Glasmatrix mit Kaffeearoma, **dadurch gekennzeichnet, dass** die Matrix aus Kaffee gewonnene Arabinogalaktane aufweist.

22. Glasmatrix nach Anspruch 21, **dadurch gekennzeichnet, dass** die aus Kaffee gewonnenen Arabinogalaktane ein durchschnittliches Molekulargewicht von mehr als ca. 10 kDa besitzen.

23. Glasmatrix nach Anspruch 21, **dadurch gekennzeichnet, dass** das durchschnittliche Molekulargewicht mehr als ca. 30 kDa beträgt.

24. Glasmatrix nach Anspruch 21, wobei die Matrix auf der Basis der gesamten festen Glasmatrix mehr als ca. 80 Gewichts-% aus Kaffee gewonnener Arabinogalaktane umfasst.

25. Glasmatrix nach einem der Ansprüche 21 bis 24, **dadurch gekennzeichnet, dass** die aus Kaffee gewonnenen Arabinogalaktane ein Gewichtsverhältnis von Galaktose zu Arabinose von über ca. 2:1 haben.

26. Glasmatrix nach Anspruch 25, **dadurch gekennzeichnet, dass** das Gewichtsverhältnis von Galaktose zu Arabinose über ca. 2,5:1 liegt.

27. Glasmatrix nach einem der Ansprüche 21 bis 25, **dadurch gekennzeichnet, dass** man die Matrix durch Mischen eines Kaffeearomas mit einer wässrigen Dispersion von aus Kaffee gewonnenen Arabinogalaktanen herstellen kann, wodurch eine Mischung gebildet wird, und man danach die hergestellte Mischung verarbeitet, so dass sie die feste Glasmatrix bildet.

28. Glasmatrix nach Anspruch 27, **dadurch gekennzeichnet, dass** die Verarbeitung das Sprühtrocknen, Sprühtrocknen und Agglomeration, Gefriertrocknen, Schmelzextrusion oder Vakuumtrocknen oder eine Kombination daraus umfasst.

29. Reine lösliche Kaffeezusammensetzung mit der Glasmatrix, wie in Anspruch 21 definiert.

30. Lösliches Kaffeegetränk nach Anspruch 29, **dadurch gekennzeichnet, dass** es weiterhin Kaffeesahne und wahlweise ein Süßungsmittel umfasst.

## Revendications

1. Procédé pour l'extraction d'arabinogalactanes à partir de café comprenant l'étape consistant à hydrolyser enzymatiquement des grains de café bruts ou torréfiés entiers ou moulus de manière à obtenir dispersion aqueuse comprenant des grains de café et des arabinogalactanes partiellement hydrolysés ; le procédé comprenant en outre les étapes suivantes :
a) traitement de la dispersion aqueuse pour obtenir une solution aqueuse comprenant des arabinogalactanes,
b) isolation des arabinogalactanes par concentration de la solution aqueuse et précipitation de l'arabinogalactane, pour ainsi obtenir une dispersion d'arabinogalactanes extraites.

2. Procédé selon la 1, comprenant en outre une ou plusieurs des étapes suivantes :
c) hydrolyse des arabinogalactanes extraites de la revendication 1 pour réduire la masse moléculaire ;
d) concentration de la dispersion aqueuse pour obtenir un liquide visqueux avec une haute concentration d'arabinogalactanes dérivées du café ;
e) séchage du précipité d'arabinogalactane de l'étape b) ou du liquide visqueux de l'étape d) pour obtenir une préparation solide d'arabinogalactanes dérivées du café.

3. Procédé selon la revendication 1, dans lequel l'hydrolyse enzymatique comprend les étapes suivantes :
- moudre les grains de café à une taille de particule moyenne comprise entre environ 10 micromètres jusqu'à environ 2 millimètres ;
- pré-traiter les grains de café moulus avec de l'eau à une température d'environ 10 °C jusqu'à environ 95 °C pendant environ 2 heures jusqu'à environ 1 semaine ;
- incuber le café moulu pré-traité à une température comprise entre environ 30 °C jusqu'à environ 80 °C avec la cellulase et la Gamanase pendant environ 12 heures jusqu'à environ 1 semaine ;
- refroidir la suspension de l'étape c) à température ambiante ;
- enlever le résidu de la suspension de l'étape c) pour obtenir une fraction soluble.

4. Procédé selon la revendication 1, dans lequel l'étape a) comprend les étapes supplémentaires suivantes :
- concentration de la solution aqueuse comprenant des arabinogalactanes à entre 5 % et 95 % de son volume d'origine ;
- centrifugation de la solution aqueuse concentrée pour obtenir une couche de surface ;
- enlèvement de la couche de surface de la solution centrifugée pour obtenir un surnageant contenant des arabinogalactanes extraites ;
- optionnellement, séchage du surnageant de l'étape b) pour obtenir un extrait en poudre enrichi en arabinogalactanes.

5. Procédé selon la revendication 1, dans lequel l'étape b) comprend les étapes supplémentaires suivantes :
- dilution du surnageant ou à dissolution de l'extrait séché enrichi en arabinogalactanes dans de l'eau pour obtenir une solution aqueuse ;
- optionnellement, dialyse de la solution aqueuse en utilisant une membrane ayant un seuil d'arrêt de masse moléculaire d'environ 5 kDa jusqu'à environ 100 kDa ;
- concentration de la solution aqueuse ou de la solution dialysée optionnellement en un liquide visqueux ;
- incubation du liquide visqueux avec environ 50 à environ 90 % en volume de solvants organiques solubles dans l'eau pour obtenir un précipité ;
- optionnellement, lavage du précipité avec des solutions d'alcool aqueuses et de l'acétone ;
- optionnellement, séchage du précipité.

6. Procédé selon l'une des revendications 1, 2, 4 et 5, dans lequel la concentration est effectuée par évaporation ou ultrafiltration.

7. Procédé selon l'une des revendications 2, 4 et 6, dans lequel le séchage est effectué en utilisant un séchage par pulvérisation, une lyophilisation, un séchage sous vide, séchage sur bande ou séchage en lit fluidisé.

8. Procédé selon l'une des revendications précédentes, **caractérisé en ce que** des arabinogalactanes sont extraites enzymatiquement de grains de café bruts entiers ou moulus.

9. Utilisation d'arabinogalactanes dérivées du café obtenues par le procédé selon l'une des revendications 1-8 comme un ingrédient alimentaire.

10. Utilisation d'arabinogalactanes dérivées du café obtenues par le procédé selon l'une des revendications 1-8 ayant une masse moyenne de masse moléculaire supérieure à environ 150 kDa, de préférence supérieure à environ 500 kDa, et plus préférablement supérieure à environ 2000 kDa comme un agent de texture dans un aliment.

11. Utilisation d'arabinogalactanes dérivées du café obtenues par le procédé selon l'une des revendications 1-8 comme un agent améliorant la viscosité dans des aliments et boissons.

12. Utilisation d'arabinogalactanes dérivées du café obtenues par le procédé selon l'une des revendications 1-8 comme un agent améliorant la sensation en bouche pour du café soluble.

13. Utilisation d'arabinogalactanes dérivées du café obtenues par le procédé selon l'une des revendications 1-8 comme un agent de texture dans des boissons.

14. Utilisation d'arabinogalactanes dérivées du café obtenues par le procédé selon l'une des revendications 1-8 comme un agent améliorant la mousse dans des aliments et boissons.

15. Utilisation d'arabinogalactanes dérivées du café obtenues par le procédé selon l'une des revendications 1-8 comme un stabilisateur de mousse dans des boissons de café.

16. Utilisation d'arabinogalactanes dérivées du café obtenues par le procédé selon l'une des revendications 1-8 comme une matrice vitreuse dans un aliment déshydraté.

17. Utilisation d'arabinogalactanes dérivées du café obtenues par le procédé selon l'une des revendications 1-8 comme une matrice pour piéger des saveurs ou arômes, ou des mélanges de ceux-ci dans un aliment déshydraté.

18. Utilisation d'arabinogalactanes dérivées du café obtenues par le procédé selon l'une des revendications 1-8 comme une matrice selon la revendication 18, **caractérisée en ce que** l'aliment déshydraté est une boisson.

19. Utilisation d'arabinogalactanes dérivées du café comme une matrice pour piéger de l'arôme de café.

20. Composition de café solide comprenant de l'arôme de café piégé dans les arabinogalactanes dérivées du café, pour laquelle l'arôme est, par rapport à du café soluble standard, perceptible de manière plus fort et de qualité préférée après un stockage prolongé.

21. Matrice vitreuse comprenant de l'arôme de café, **caractérisée en ce que** la matrice comprend des arabinogalactanes dérivées du café.

22. Matrice vitreuse selon la revendication 21, **caractérisée en ce que** les arabinogalactanes dérivées du café ont une masse moléculaire moyenne supérieure à environ 10 kDa.

23. Matrice vitreuse selon la revendication 21, **caractérisée en ce que** la masse moléculaire moyenne est supérieure à environ 30 kDa.

24. Matrice vitreuse selon la revendication 21, la matrice comprenant plus qu'environ 80 % en poids d'arabinogalactanes dérivées du café sur la base de la matrice vitreuse solide totale.

25. Matrice vitreuse selon l'une des revendications 21 à 24, **caractérisée en ce que** les arabinogalactanes dérivées du café ont un rapport en poids de galactose par rapport à arabinose supérieur à environ 2:1.

26. Matrice vitreuse selon la revendication 25, **caractérisée en ce que** le rapport en poids de galactose par rapport à arabinose est supérieur à environ 2,5:1.

27. Matrice vitreuse selon l'une des revendications 21 à 25, **caractérisée en ce que** la matrice est obtenue par le mélange d'un arôme de café avec une dispersion aqueuse d'arabinogalactanes dérivées du café, formant ainsi un mélange, et ensuite par le traitement du mélange obtenu pour former la matrice vitreuse solide.

28. Matrice vitreuse selon la revendication 27, **caractérisée en ce que** le traitement comprend un séchage par pulvérisation, un séchage par pulvérisation et une agglomération, une lyophilisation, une extrusion à l'état fondu, ou un séchage sous vide, ou une combinaison de ceux-ci.

29. Composition de café soluble pure comprenant la matrice vitreuse telle que définie dans la revendication 21.

30. Composition de boisson de café soluble selon la revendication 29, **caractérisée en ce qu'**elle comprend en outre un colorant à café et optionnellement un édulcorant.
